(19) Europäisches Patentamt
European Patent Office
Office européen des brevets

(11) EP 3 714 868 A1

(12) EUROPEAN PATENT APPLICATION
published in accordance with Art. 153(4) EPC

(43) Date of publication:
**30.09.2020 Bulletin 2020/40**

(21) Application number: **18877776.7**

(22) Date of filing: **19.11.2018**

(51) Int Cl.:
***A61K 8/49*** *(2006.01)* ***A61K 8/46*** *(2006.01)*
***A61K 8/81*** *(2006.01)* ***A61Q 5/02*** *(2006.01)*
***A61Q 5/10*** *(2006.01)*

(86) International application number:
**PCT/JP2018/042680**

(87) International publication number:
**WO 2019/098367 (23.05.2019 Gazette 2019/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.11.2017 JP 2017223219**

(71) Applicant: **Kao Corporation**
**Chuo-Ku**
**Tokyo 103-8210 (JP)**

(72) Inventors:
- **HIRAYAMA, Masutaro**
  **Wakayama-shi**
  **Wakayama 640-8580 (JP)**
- **YOSHIDA, Hiroshi**
  **Tokyo 131-8501 (JP)**
- **SAKAI, Yuta**
  **Tokyo 131-8501 (JP)**

(74) Representative: **Hoffmann Eitle**
**Patent- und Rechtsanwälte PartmbB**
**Arabellastraße 30**
**81925 München (DE)**

# (54) COSMETIC HAIR PREPARATION

(57) The present invention relates to a hair cosmetic containing the following components (A) and (B):
(A) a compound represented by the following general formula (1) or a salt thereof:

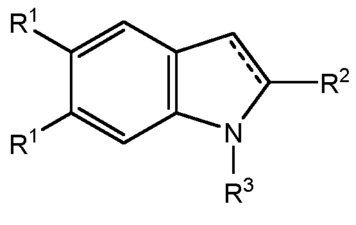

(1)

wherein a broken line represents the presence or absence of a π bond; $R^1$ represents a hydroxy group or an acetoxy group; $R^2$ represents a hydrogen atom or -COOR (where R is a hydrogen atom, a methyl group, or an ethyl group); and $R^3$ represents a hydrogen atom, an acetyl group, a methyl group, or an ethyl group, and
(B) at least one polymer selected from the group consisting of polyvinyl alcohol and polyvinylpyrrolidone each having an average degree of polymerization of 700 or more and 10,000 or less.

EP 3 714 868 A1

## Description

Field of the Invention

The present invention relates to a hair cosmetic.

Background of the Invention

Conventionally, for gray hair dyeing, it is general to use a hair dye, such as a two-pack type hair color and a one-pack type hair manicure. However, as for a hair dyeing treatment with such a hair dye, due to concerns about necessity of shampooing and soil of the surrounding area, and so on, not only a place at which the hair dyeing is carried out is restricted, but also the treatment method is complicated. In addition, there were also involved problems, such as unnaturalness just after hair dyeing owing to an abrupt change of color of hair by the hair dyeing treatment, and generation of a level difference in color between the hair in a portion where the hair dyeing treatment has been performed and the gray hair which have newly grown.

In contrast, as a hair cosmetic which covers a hair surface with a melanin polymer through a daily hair care behavior, such as styling, to enable one to simply obscure gray hair and to dye the hair by continuous employments, a non-washed away type hair cosmetic containing a 5,6-dihydroxyindole derivative and a film-forming polymer is proposed (see PTL 1).

Meanwhile, as for a dyeing method of hair, there is known a method in which a treatment with a composition containing a transition metal ion and a treatment with a composition containing 5,6-dihydroxyindole-2-carboxylic acid are sequentially performed to promote conversion of the foregoing 5,6-dihydroxyindole derive into a melanin pigment (see PTL 2).

Citation List

Patent Literature

PTL 1: JP 2007-326805 A
PTL 2: JP 01-319413 A

Summary of the Invention

The present invention relates to the following [1] to [2].

[1] A hair cosmetic containing the following components (A) and (B):

(A) a compound represented by the following general formula (1) or a salt thereof:

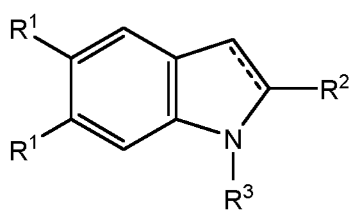 (1)

wherein a broken line represents the presence or absence of a $\pi$ bond; $R^1$ represents a hydroxy group or an acetoxy group; $R^2$ represents a hydrogen atom or -COOR (where R is a hydrogen atom, a methyl group, or an ethyl group); and $R^3$ represents a hydrogen atom, an acetyl group, a methyl group, or an ethyl group, and
(B) at least one polymer selected from the group consisting of polyvinyl alcohol and polyvinylpyrrolidone each having an average degree of polymerization of 700 or more and 10,000 or less.

[2] A dyeing method of hair including a step of applying the hair cosmetic as set forth in the above [1] on hair.

Detailed Description of the Invention

[Hair Cosmetic]

The hair cosmetic of the present invention contains the following components (A) and (B):

(A) a compound represented by the following general formula (1) or a salt thereof:

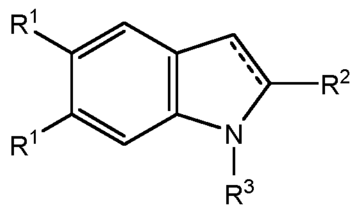
(1)

wherein a broken line represents the presence or absence of a $\pi$ bond; $R^1$ represents a hydroxy group or an acetoxy group; $R^2$ represents a hydrogen atom or -COOR (where R is a hydrogen atom, a methyl group, or an ethyl group); and $R^3$ represents a hydrogen atom, an acetyl group, a methyl group, or an ethyl group, and

(B) at least one polymer selected from the group consisting of polyvinyl alcohol and polyvinylpyrrolidone each having an average degree of polymerization of 700 or more and 10,000 or less.

Although the hair cosmetic described in PTL 1 can simply obscure gray hair through a daily hair care behavior, hair dyeing properties were not sufficiently satisfactory. In addition, in the method described in PTL 2, not only the operations were complicated, but also a high-degree treatment technique was required.

A problem of the present invention is to provide a hair cosmetic which is able to simply obscure gray hair through a daily hair care behavior, such as shampooing, and to dye the hair to a sufficient level by continuous employments.

The present inventors have found that in a hair cosmetic, the aforementioned problem can be solved by using a combination of a predetermined melanin precursor with at least one polymer selected from the group consisting of polyvinyl alcohol and polyvinylpyrrolidone each having a specified degree of polymerization. In accordance with the hair cosmetic of the present invention, it is able to simply obscure gray hair through a daily hair care behavior, such as shampooing, and to dye the hair to a sufficient level by continuous employments.

In the present invention, examples of the hair cosmetic include a hair cleansing agent, such as a shampoo, and besides, a hair rinse, a hair conditioning agent, a hair treatment agent, a hair styling agent, and a hair growth promoter. Of these, a hair cleansing agent is preferred from the viewpoint of obtaining the effects of the present invention. The formulation of the hair cosmetic is not particularly limited, and it is possible to take an arbitrary formulation, such as a liquid, a foam, a paste, a cream, a solid, and a powder. For example, in the case of a hair cleansing agent, its formulation is preferably a liquid, a paste, or a cream, and more preferably a liquid.

By daily using the hair cosmetic containing the aforementioned component (A) and component (B), even when a hair dye is not used, it is able to simply obscure the gray hair through a daily hair care behavior, such as shampooing, and to obtain high hair dyeing properties.

The component (A) which is used in the present invention is a melanin precursor that penetrates into the interior of hair and is polymerized through air oxidation to form a melanin pigment, thereby enabling one to dye gray hair in black hair. However, the component (A) is liable to cohere in water, for example, during use of the hair cosmetic, and therefore, it hardly penetrates into the hair and could not sufficiently perform hair dyeing.

In addition, there was involved such a problem that a plenty of the component (A) is rinsed away during hair cleansing.

In the present invention, by using a combination of the component (A) that is the melanin precursor with the component (B) having an average degree of polymerization falling within a predetermined range, for example, dispersibility of the component (A) is improved even in water, and cohesion is suppressed, whereby penetration into the hair is promoted. For that reason, an effect for allowing the component (A) to remain in the hair is brought, so that it may be considered that the hair dyeing properties are improved.

Although a mechanism in which polyvinyl alcohol (PVA) and polyvinylpyrrolidone (PVP) each serving as the component (B) improve the dispersibility of the component (A) is not elucidated yet, it may be considered that in view of the fact that a hydrophobic main chain which PVA has and the component (A) interact with each other, cohesion of the component (A) can be suppressed; and that in view of the fact that PVA has a hydrophilic hydroxy group, solubility of the component (A) which interacts with PVA can be maintained. Similar to PVA, in view of the fact that PVP has a hydrophobic main chain and a hydrophilic side chain, it may be conjectured that an effect for suppressing cohesion of

the component (A) is brought by means of the same mechanism as mentioned above. From such a cohesion-suppressing effect, it may be considered that an effect for allowing the component (A) to remain in the hair during use of the hair cosmetic (for example, during hair cleansing) is brought, too.

<Component (A)>

The hair cosmetic of the present invention contains the component (A) that is the compound represented by the following general formula (1) or a salt thereof. The component (A) is a melanin precursor which is polymerized through air oxidation and converted into a melanin pigment, and it acts as a dyeing agent of hair.

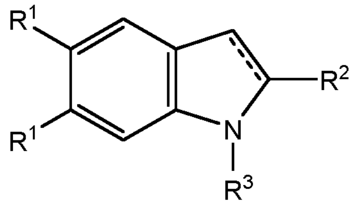

(1)

In the formula, a broken line represents the presence or absence of a $\pi$ bond; $R^1$ represents a hydroxy group or an acetoxy group; $R^2$ represents a hydrogen atom or -COOR (where R is a hydrogen atom, a methyl group, or an ethyl group); and $R^3$ represents a hydrogen atom, an acetyl group, a methyl group, or an ethyl group.

The melanin precursor of the component (A) is an indole derivative or an indoline derivative represented by the general formula (1), or a salt thereof, and in the present invention, one or a combination of two or more thereof can be used. The component (A) is more preferably an indole derivative (namely, a $\pi$ bond exists in the broken line portion in the general formula (1)) from the viewpoint of hair dyeing properties.

From the viewpoint of availability and hair dyeing properties of the component (A), in the general formula (1), $R^1$ is preferably a hydroxy group; $R^2$ is preferably a hydrogen atom or -COOR (where R is a hydrogen atom, a methyl group, or an ethyl group), and more preferably a hydrogen atom or -COOH; and $R^3$ is preferably a hydrogen atom.

Examples of the compound represented by the general formula (1) include 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid.

Examples of the salt of the compound represented by the general formula (1) include a hydrochloride, a hydrobromide, a sulfate, a phosphate, an acetate, a propionate, a lactate, and a citrate of the foregoing compounds. Above all, a hydrobromide is preferred from the viewpoint of availability.

In the general formula (1), when $R^2$ is -COOH, examples of the salt of the compound represented by the general formula (1) include carboxylates thereof ($R^2$ is $-COO^-X^+$ ($X^+$ is a cation, such as an alkali metal ion, e.g., $Na^+$ and $K^+$, an alkaline earth metal ion, e.g., $Ca^+$ and $Mg^+$, and an ammonium ion)).

From the viewpoint of dyeing hair in a natural color shade, the component (A) is preferably one or more selected from the group consisting of 5,6-dihydroxyindole, 5,6-dihydroxyindole-2-carboxylic acid, 5,6-dihydroxyindoline, and 5,6-dihydroxyindoline-2-carboxylic acid, and salts thereof; more preferably one or more selected from the group consisting of 5,6-dihydroxyindole, 5,6-dihydroxyindole-2-carboxylic acid, and 5,6-dihydroxyindoline, and salts thereof; still more preferably one or more selected from the group consisting of 5,6-dihydroxyindole, 5,6-dihydroxyindole-2-carboxylic acid, and 5,6-dihydroxyindoline hydrobromide; yet still more preferably one or two selected from the group consisting of 5,6-dihydroxyindole and 5,6-dihydroxyindole-2-carboxylic acid; and even yet still more preferably a combination of 5,6-dihydroxyindole and 5,6-dihydroxyindole-2-carboxylic acid.

In the case of use of a combination of 5,6-dihydroxyindole and 5,6-dihydroxyindole-2-carboxylic acid, a molar ratio thereof is preferably in a range of 50/50 to 99/1, more preferably in a range of 80/20 to 99/1, and still more preferably in a range of 85/15 to 95/5. When the molar ratio of 5,6-dihydroxyindole and 5,6-dihydroxyindole-2-carboxylic acid falls within the aforementioned range, finish of the hair after hair dyeing becomes close to a natural color tint.

The molar ratio of 5,6-dihydroxyindole and 5,6-dihydroxyindole-2-carboxylic acid can be quantitatively determined by means of reversed phase HPLC.

From the viewpoint of improvement in hair dyeing properties, the content of the component (A) in the hair

cosmetic is preferably 0.01% by mass or more, more preferably 0.05% by mass or more, still more preferably 0.10% by mass or more, and yet still more preferably 0.12% by mass or more. In addition, from the viewpoint of improvement in hair dyeing properties, blending stability, operability, and economy, the foregoing content of the component (A) is preferably 40% by mass or less, more preferably 10% by mass or less, still more preferably 5% by mass or less, yet still more preferably 2% by mass or less, even yet still more preferably 1% by mass or less, and even still more preferably 0.5% by mass or less.

<Component (B)>

The hair cosmetic of the present invention contains at least one polymer component (B) selected from the group consisting of polyvinyl alcohol and polyvinylpyrrolidone each having an average degree of polymerization of 700 or more and 10,000 or less. In the hair cosmetic, by using a composition of the component (A) with the component (B), on blending in a hair cleansing agent, for example, a shampoo, dispersibility of the component (A) is improved even in water, and cohesion is suppressed, whereby penetration into the hair is promoted, and therefore, high hair dyeing properties can be obtained. Above all, from the viewpoint of improvement in hair dyeing properties, it is preferred that the component (B) is polyvinyl alcohol.

An average degree of polymerization of the component (B) is 700 or more and 10,000 or less. When the average degree of polymerization of the component (B) is 700 or more, an effect for improving the hair dyeing properties is favorable, whereas when it is 10,000 or less, solubility in the hair cosmetic is favorable.

From the viewpoint of improvement in hair dyeing properties, the average degree of polymerization of the component (B) is preferably 800 or more, more preferably 1,000 or more, still more preferably 1,200 or more, and yet still more preferably 1,800 or more. On the other hand, from the viewpoint of solubility in the hair cosmetic, the foregoing average degree of polymerization is preferably 7,000 or less, more preferably 3,700 or less, still more preferably 3,600 or less, yet still more preferably 2,600 or less, even yet still more preferably 2,450 or less, and even still more preferably 2,300 or less.

As the component (B), two or more polymers may be used. In that case, the average degree of polymerization of the component (B) is determined by calculating a weighted average from the average degrees of polymerization and blending amounts of the respective polymers.

In the case where the component (B) is polyvinyl alcohol, though a degree of saponification of polyvinyl alcohol is not particularly limited, it is preferably 70% or more, and it is preferably 95% or less, and more preferably 90% or less, from the viewpoint of improvement in hair dyeing properties and handling properties during blending in the hair cosmetic.

From the viewpoint of improvement in hair dyeing properties, the content of the component (B) in the hair cosmetic is preferably 0.05% by mass or more, more preferably 0.15% by mass or more, still more preferably 0.20% by mass or more, and yet still more preferably 0.25% by mass or more in terms of the content in the hair cosmetic. On the other hand, from the viewpoint of improvement in blending stability, the foregoing content is preferably 5.0% by mass or less, more preferably 3.0% by mass or less, still more preferably 1.0% by mass or less, and yet still more preferably 0.5% by mass or less.

From the viewpoint of improvement in hair dyeing properties, a mass ratio (B)/(A) of the component (B) to the component (A) in the hair cosmetic is preferably 0.001 or more, more preferably 0.01 or more, still more preferably 0.1 or more, yet still more preferably 0.5 or more, and even yet still more preferably 1 or more. On the other hand, from the viewpoint of improvement in blending stability, the mass ratio (B)/(A) of the component (B) to the component (A) is preferably 1,000 or less, more preferably 200 or less, still more preferably 100 or less, and yet still more preferably 5 or less.

<Component (C): Surfactant>

From the viewpoint of improvement in hair dyeing properties and the viewpoint of effectively revealing the effects as the hair cosmetic, it is preferred that the hair cosmetic of the present invention further contains a surfactant as a component (C). Examples of the surfactant include an anionic surfactant, a cationic surfactant, an ampholytic surfactant, and a nonionic surfactant. In the case where the hair cosmetic is a hair cleansing agent, it preferably contains at least an anionic surfactant as the component (C), and in the case where the hair cosmetic is a hair rinse or a hair conditioning agent, it preferably contains at least a cationic surfactant as the component (C). Furthermore, at least one surfactant selected from the group consisting of an ampholytic surfactant and a nonionic surfactant may be used in combination therewith.

(Anionic Surfactant)

In the case where the hair cosmetic is a hair cleansing agent, it preferably contains an anionic surfactant. Examples of the anionic surfactant include an alkylbenzene sulfonate, an alkyl or alkenyl ether sulfate, an alkyl or alkenyl

sulfate, an olefin sulfonate, an alkane sulfonate, a saturated or unsaturated fatty acid salt, an alkyl or alkenyl ether carboxylate, an $\alpha$-sulfo fatty acid salt, a N-acylamino acid, a phosphoric mono- or diester, and a sulfosuccinic acid ester. One or more of these anionic surfactants can be used.

Examples of a counter ion of an anionic group of the anionic surfactant include an alkali metal ion, such as a sodium ion and a potassium ion; an alkaline earth metal ion, such as a calcium ion and a magnesium ion; an ammonium ion; and an alkanolamine having 1 to 3 alkanol groups having 2 or 3 carbon atoms (for example, monoethanolamine, diethanolamine, triethanolamine, and triisopropanolamine).

Above all, one or more selected from the group consisting of an alkyl ether sulfate and an alkyl ether carboxylate are preferred from the viewpoint of favorable lathering, easiness for washing, and an effect for improving the hair dyeing properties in the case where the hair cosmetic is a hair cleansing agent. Examples of the alkyl ether sulfate include a polyoxyethylene alkyl ether sulfate, and examples of the alkyl ether carboxylate include a polyoxyethylene alkyl ether acetate.

In the case where the hair cosmetic contains an anionic surfactant as the component (C), its content in the hair cosmetic is preferably 3% by mass or more, more preferably 5% by mass or more, and still more preferably 8% by mass or more from the viewpoint of favorable lathering and easiness for washing in the case where the hair cosmetic is a hair cleansing agent. In addition, the foregoing content is preferably 40% by mass or less, more preferably 30% by mass or less, still more preferably 20% by mass or less, and yet still more preferably 15% by mass or less from the viewpoint of improvement in hair dyeing properties and suppressing any damage to the hair.

(Cationic Surfactant)

Examples of the cationic surfactant include a mono- or di-long chain alkyl quaternary ammonium salt represented by the following general formula.

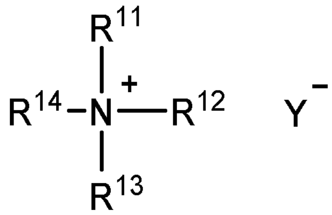

In the formula, $R^{11}$ represents a linear or branched alkyl group having 8 or more and 22 or less carbon atoms, or a group represented by $R^{15}CONH(CH_2)_m$-, $R^{15}$-O-$(CH_2)_m$-, or $R^{15}COO(CH_2)_m$-($R^{15}$ represents a linear or branched alkyl group having 7 or more and 21 or less carbon atoms, and m represents a number of 1 or more and 4 or less); $R^{12}$ represents a linear or branched alkyl group having 1 or more and 22 or less carbon atoms, or a group represented by the aforementioned $R^{15}CONH(CH_2)_m$-, $R^{15}$-O-$(CH_2)_m$-, or $R^{15}COO(CH_2)_m$-; $R^{13}$ and $R^{14}$ each independently represent an alkyl group having 1 or more and 4 or less carbon atoms; and $Y^-$ represents a chloride ion, a bromide ion, or a methosulfate ion.

As specific examples of the cationic surfactant, from the viewpoint of imparting an excellent touch to hair, one or more selected from the group consisting of a monoalkyltrimethylammonium chloride, a dialkyldimethylammonium chloride, a monoalkyloxyalkyltrimethylammonium chloride, and a monoalkyltrimethylammonium bromide are preferred. Above all, one or more selected from the group consisting of behenyltrimethylammonium chloride, stearyltrimethylammonium chloride (steartrimonium chloride), cetyltrimethylammonium chloride (cetrimonium chloride), lauryltrimethylammonium chloride (lauryltrimonium chloride), a dialkyl(C12-C18)dimethylammonium chloride, and octadecyloxypropyltrimethylammonium chloride are more preferred.

In the case where the hair cosmetic contains a cationic surfactant as the component (C), from the viewpoint of imparting an excellent touch to hair, its content in the hair cosmetic is preferably 0.01% by mass or more, and more preferably 0.05% by mass or more, and from the viewpoint of suppressing a lowering of touch to hair, the foregoing content is preferably 10% by mass or less, and more preferably 5% by mass or less.

(Ampholytic Surfactant)

Examples of the ampholytic surfactant include a betaine-based surfactant, such as an alkyl dimethyl amino acetic acid betaine, a fatty acid amidopropylbetaine, and an alkylhydroxy sulfobetaine; and a sultaine-based surfactant, such as lauryl hydroxysultaine. Above all, a betaine-based surfactant is preferred, and a fatty acid amidopropylbetaine is more preferred. The fatty acid amidopropylbetaine is preferably one having an acyl group having 8 or more and 18 or less carbon atoms, and moreover, 10 or more and 16 or less carbon atoms, and one or more selected from the group

consisting of lauric acid amidopropylbetaine, a palm kernel oil fatty acid amidopropylbetaine, and a coconut oil fatty acid amidopropylbetaine are preferred.

In the case where the hair cosmetic contains an ampholytic surfactant as the component (C), from the viewpoint of adaptability to hair and favorable lathering, its content in the hair cosmetic is preferably 0.05% by mass or more, more preferably 0.1% by mass or more, still more preferably 0.15% by mass or more, yet still more preferably 0.50% by mass or more, and even yet still more preferably 1.0% by mass or more, and it is preferably 15% by mass or less, more preferably 12% by mass or less, and still more preferably 10% by mass or less.

(Nonionic Surfactant)

Examples of the nonionic surfactant include a polyoxyalkylene alkyl ether, a polyoxyalkylene alkenyl ether, a polyoxyalkylene fatty acid ester, a higher fatty acid sucrose ester, a polyglycerin fatty acid ester, a polyoxyethylene hydrogenated castor oil, a polyoxyethylene sorbitan fatty acid ester, a polyoxyethylene sorbitol fatty acid ester, an alkyl saccharide, an alkylamine oxide, and an alkylamidoamine oxide. Of these, one or more selected from the group consisting of a polyoxyalkylene alkyl ether, a polyoxyethylene hydrogenated castor oil, and an alkyl saccharide are preferred, and a polyoxyalkylene alkyl ether is more preferred.

In the case where the hair cosmetic contains a nonionic surfactant as the component (C), its content in the hair cosmetic is preferably 0.01% by mass or more, and more preferably 0.02% by mass or more, and from the viewpoint of suppressing a lowering of touch to hair, the foregoing content is preferably 5% by mass or less, and more preferably 3% by mass or less.

<Oil (D)>

From the viewpoint of improvement in touch to hair, the viewpoint of improvement in hair dyeing properties, and the viewpoint of effectively revealing an effect as the hair cosmetic, the hair cosmetic of the present invention can further contain an oil as a component (D).

Examples of the oil include hydrocarbons, such as squalene, squalane, liquid paraffin, liquid isoparaffin, and cycloparaffin; glycerides, such as castor oil, cacao oil, mink oil, avocado oil, and olive oil; waxes, such as beeswax, spermaceti wax, lanolin, and carnauba wax; esters, such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids, such as capric acid, lauric acid, myristic acid, palmitic acid, stearic acid, behenic acid, oleic acid, coconut oil fatty acid, isostearic acid, and isopalmitic acid; and besides, isostearyl glyceryl ether and polyoxypropylene butyl ether. The oil can be used alone or in combination of two or more thereof.

In the case of using the oil, its content in the hair cosmetic is preferably 0.5% by mass or more, more preferably 1% by mass or more, and still more preferably 2% by mass or more from the viewpoint of improvement in touch to hair, the viewpoint of improvement in hair dyeing properties, and the viewpoint of cleansing properties in the case where the hair cosmetic is a hair cleansing agent; and the foregoing content is preferably 10% by mass or less, more preferably 5% by mass or less, and still more preferably 3% by mass or less from the viewpoint of finger-combing smoothness during hair rinsing and blending stability.

<Alkaline Agent>

From the viewpoint of improvement in hair dyeing properties, the hair cosmetic of the present invention can further contain an alkaline agent. The alkaline agent has not only an action to swell hair, thereby opening the cuticle and penetrating the dyeing agent component, such as the component (A), into the interior of the hair, but also an action to promote a polymerization reaction of the component (A), thereby improving the hair dyeing properties.

As the alkaline agent, any material can be used without particular limitations so long as it is an alkaline agent that is used for usual hair dyes. Examples of the alkaline agent include ammonia; alkanolamines, such as mono-, di-, or tri-methanolamine and mono-, di-, or tri-ethanolamine; alkylamines, such as methylamine, dimethylamine, ethylamine, diethylamine, N-methylethylamine, propylamine, and butylamine; aralkylamines, such as benzylamine; and inorganic alkali compounds, such as sodium hydroxide and potassium hydroxide, and one or more of these materials can be used. The carbon number of the alkanolamine, alkylamine, or aralkylamine is preferably 10 or less, and more preferably 8 or less from the viewpoint of water solubility.

Above all, from the viewpoint of hair dyeing properties, the component (B) is preferably one or more selected from the group consisting of ammonia, an alkanolamine, an alkylamine, an aralkylamine, sodium hydroxide, and potassium hydroxide. The component (B) more preferably contains one or more of ammonia and an alkanolamine, still more preferably contains a monoalkanolamine, and yet still more preferably contains monoethanolamine.

The content of the alkaline agent in the hair cosmetic is preferably 0.01% by mass or more, more preferably

0.1% by mass or more, and still more preferably 0.5% by mass or more from the viewpoint of obtaining high hair dyeing properties, and it is preferably 10% by mass or less, more preferably 5% by mass or less, and still more preferably 3% by mass or less from the viewpoint of suppressing irritation.

<Buffering Agent>

From the viewpoint of improving a pH buffering ability, the hair cosmetic of the present invention can further contain a buffering agent. In view of the fact of containing the buffering agent, for example, in the case where the hair cosmetic is diluted with water during use, in order that the component (A) may reveal high hair dyeing properties, it is easy to maintain an optimum pH range.

Although the buffering agent is not particularly limited so long as it has a pH buffering action, since the component (A) that is the melanin precursor reacts with oxygen in air under a basic condition, whereby it is liable to be converted into a melanin pigment, a buffering agent capable of regulating the pH of the hair cosmetic to the basic condition is preferred.

From the aforementioned viewpoint, as for the buffering agent, a buffering agent containing, as a basic component, any one of a carbonate, such as sodium carbonate, potassium carbonate, sodium bicarbonate, and potassium bicarbonate, glycine, sodium tetraborate, and ammonium chloride, or a buffering agent composed of a combination of two or more basic components, such as sodium carbonate-sodium bicarbonate, is preferred; a buffering agent containing a carbonate is more preferred; and a buffering agent containing sodium bicarbonate is still more preferred.

The buffering agent may further contain a protonating agent as a buffering agent component. The protonating agent may be any of a monobasic acid and a polybasic acid, and may be any of an organic acid (the carbon number is 1 or more and 8 or less, provided that ascorbic acid is excluded) and an inorganic acid. As the protonating agent, one or more selected from the group consisting of hydrochloric acid, sulfuric acid, phosphoric acid, formic acid, acetic acid, and citric acid are exemplified, and one or two selected from the group consisting of phosphoric acid and citric acid are more preferred.

From the viewpoint of improvement in hair dyeing properties, the buffering agent component constituting the buffering agent is still more preferably composed of a combination of sodium bicarbonate with one or two selected from the group consisting of phosphoric acid and citric acid.

Although the content of the buffering agent in the hair cosmetic is not particularly limited so long as it is an amount at which the pH of the hair cosmetic can be regulated to a desired range, it is preferably 0.5% by mass or more, more preferably 1.0% by mass or more, and still more preferably 1.5% by mass or more from the viewpoint of improving a pH buffering ability in the case where the hair cosmetic is diluted with water. In addition, from the viewpoint of formulation stability, the content of the buffering agent in the hair cosmetic is preferably 5.0% by mass or less, more preferably 4.0% by mass or less, and still more preferably 3.5% by mass or less.

The content of the buffering agent means a sum total of the active components of the buffering agent components constituting the buffering agent.

<Other Components>

The hair cosmetic of the present invention may appropriately contain, in addition to the aforementioned components, a component which is usually used for hair cosmetics or hair dyes, within a range where the purpose of the present invention is not impaired. Examples of the foregoing component include an antioxidant, a silicone, a higher alcohol, an aromatic alcohol, a dyeing agent other than the component (A), a polymer other than the component (B), an anti-dandruff agent, a vitamin compound, a disinfectant, an antiinflammatory agent, an antiseptic, a chelating agent, a humectant, a pearlescent agent, a ceramide, a perfume, and an ultraviolet absorber.

(Antioxidant)

Examples of the antioxidant include sulfurous acid, ascorbic acid, thioglycolic acid, L-cysteine, and N-acetyl-L-cysteine, and salts thereof. From the viewpoint of stabilization of the component (A) and improvement in dyeing properties, ascorbic acid and a salt thereof are preferred.

In the case of using the antioxidant, its content in the hair cosmetic is preferably 0.05% by mass or more, and more preferably 0.1% by mass or more, and it is preferably 10% by mass or less, more preferably 5% by mass or less, and still more preferably 2% by mass or less.

(Silicone)

For example, in the case where the hair cosmetic is a hair cleansing agent, the silicone has an effect for

improving foam texture, foam slipperiness, crease reduction during cleansing, and smoothness during drying. Examples of the silicone include dimethyl polysiloxane; methylphenyl polysiloxane; an amino-modified silicone, such as amodimethicone, aminoethylaminopropyl dimethicone, and aminopropyl dimethicone; a cyclic silicone; a polyether-modified silicone; a fatty acid-modified silicone; an alcohol-modified silicone; an alkoxy-modified silicone; an epoxy-modified silicone; a fluorine-modified silicone; and an alkyl-modified silicone. One or more of these silicones can be contained.

Above all, dimethyl polysiloxane is preferred as the silicone from the same viewpoint as mentioned above.

In the case of using the silicone, its content in the hair cosmetic is preferably 0.1% by mass or more, more preferably 0.2% by mass or more, still more preferably 0.5% by mass or more, and yet still more preferably 2% by mass or more from the same viewpoint as mentioned above, and it is preferably 15% by mass or less, more preferably 10% by mass or less, and still more preferably 8% by mass or less from the same viewpoint as mentioned above and the viewpoint of economy.

(Higher Alcohol)

The hair cosmetic of the present invention can contain a higher alcohol. In the case where the hair cosmetic is a hair cosmetic selected from the group consisting of a hair rinse, a hair conditioning agent, and a hair treatment agent, emulsification stability is improved, and in the case where the hair cosmetic is a hair cleansing agent, there is brought an effect, such as improvement in lathering.

As the higher alcohol, one represented by a general formula: $R^{21}$-OH [wherein $R^{21}$ represents a linear or branched hydrocarbon group having 12 or more and 24 or less carbon atoms] can be used. $R^{21}$ is preferably a linear or branched aliphatic hydrocarbon group having 12 or more and 24 or less carbon atoms; more preferably a linear or branched alkyl group having 12 or more and 24 or less carbon atoms or a linear or branched alkenyl group having 12 or more and 24 or less carbon atoms; and still more preferably a linear alkyl group having 12 or more and 24 or less carbon atoms or a linear alkenyl group having 12 or more and 24 or less carbon atoms.

Examples of the higher alcohol include lauryl alcohol, myristyl alcohol, cetyl alcohol, stearyl alcohol, isostearyl alcohol, arachidyl alcohol, behenyl alcohol, carnaubyl alcohol, and oleyl alcohol. The higher alcohol can be used alone or in combination of two or more thereof.

From the viewpoint of controlling a change in viscosity with a lapse of time of the hair cosmetic, the higher alcohol is preferably a combination of (Ia) one in which $R^{21}$ in the aforementioned general formula is a linear alkyl group having 12 or more and 18 or less carbon atoms with (Ib) one in which $R^{21}$ is a linear alkyl group having 20 or more and 24 or less carbon atoms; and more preferably a combination of (Ia) one in which $R^{21}$ is a linear alkyl group having 14 or more and 18 or less carbon atoms with (Ib) one in which $R^{21}$ is a linear alkyl group having 20 or more and 22 or less carbon atoms. From the viewpoint of controlling a change in viscosity with a lapse of time, a mass ratio of the component (Ia) to the component (Ib) [(Ia)/(Ib)] is preferably 5/95 to 95/5, more preferably 10/90 to 90/10, still more preferably 15/85 to 85/15, and yet still more preferably 20/80 to 80/20.

In the case where the hair cosmetic contains the higher alcohol, its content is preferably 0.1% by mass or more, more preferably 0.5% by mass or more, and still more preferably 1% by mass or more, and it is preferably 20% by mass or less, more preferably 15% by mass or less, and still more preferably 10% by mass or less, from the viewpoint of improvement in emulsification stability.

(Aromatic Alcohol)

From the viewpoint of solubility of the polymer component, the hair cosmetic of the present invention may further contain an aromatic alcohol. Examples of the aromatic alcohol include benzyloxyethanol, benzyl alcohol, phenethyl alcohol, $\gamma$-phenylpropyl alcohol, cinnamic alcohol, anise alcohol, p-methylbenzyl alcohol, $\alpha,\alpha$-dimethylphenethyl alcohol, $\alpha$-phenyl ethanol, and phenoxyethanol. Of these, one or more selected from the group consisting of benzyloxyethanol and benzyl alcohol are preferred, and benzyl alcohol is more preferred.

In the case of using the aromatic alcohol, its content in the hair cosmetic is preferably 0.05% by mass or more, and more preferably 0.1% by mass or more, and it is preferably 5% by mass or less, more preferably 3% by mass or less, and still more preferably 2% by mass or less, from the same viewpoint as mentioned above.

(Dyeing Agent Other Than Component (A))

The hair cosmetic of the present invention may further contain a dyeing agent other than the component (A). Examples of the foregoing dyeing agent include an oxidation dye (constituted of a precursor and a coupler) and a direct dye, each of which is typically used for hair dyes.

Examples of the precursor in the oxidation dye include one or more selected from the group consisting of paraphenylenediamine, toluene-2,5-diamine, 2-chloro-parapheylenediamine, N-methoxyethylparaphenylenediamine,

N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3-bis(N-(2-hydroxyethyl)-N-(4-aminophenyl)amino)-2-propanol, PEG-3,2,2'-paraphenylenediamine, paraminophenol, paramethylaminophenol, 3-methyl-4-aminophenol [= 4-aminometacresol], 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraminopyrimidine, 2,5,6-triamino-4-hydroxypyrimidine, 4,5-diamino-1-(4'-chlorobenzyl)pyrazole, and 1-hydroxyethyl-4,5-diaminopyrazole, and salts thereof.

Examples of the coupler in the oxidation dye include one or more selected from the group consisting of metaphenylenediamine, 2,4-diaminophenoxyethanol, 2-amino-4-(2-hydroxyethylamino)anisole [= 2-amino-4-(β-hydroxyethyl)aminoanisole], 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaminophenol, 2-methyl-5-aminophenol [= 5-aminoorthocresol], 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, and 2,6-diaminopyridine, and salts thereof.

Examples of the direct dye include an acidic dye, a nitro dye, a disperse dye, a basic dyes, and a direct dye described in JP 2003-342139 A.

Examples of the acidic dye include Blue No. 1, Violet No. 401, Black No. 401, Orange No. 205, Red No. 227, Red No. 106, Yellow No. 203, and Acidic Orange 3. Examples of the nitro dye include 2-nitroparaphenylenediamine, 2-amino-6-chloro-4-nitrophenol, 3-nitro-p-hydroxyethylaminophenol, 4-nitroorthophenylenediamine, 4-amino-3-nitrophenol, 4-hydroxypropylamino-3-nitrophenol, HC Blue 2, HC Orange 1, HC Red 1, HC Yellow 2, HC Yellow 4, HC Yellow 5, HC Red 3, and N,N-bis-(2-hydroxyethyl)-2-nitroparaphenylenediamine. Examples of the disperse dye include Disperse Violet 1, Disperse Blue 1, and Disperse Black 9. Examples of the basic dye include Basic Blue 99, Basic Brown 16, Basic Brown 17, Basic Red 76, Basic Red 51, Basic Yellow 57, Basic Yellow 87, and Basic Orange 31. These direct dyes can be used alone or in combination of two or more thereof.

The aforementioned dyeing agent other than the component (A) can be used alone or in combination of two or more thereof. As the foregoing dyeing agent, the acidic dye is preferred; as the precursor, paraphenylenediamine, toluene-2,5-diamine, paraminophenol, 4-aminometacresol, and 1-hydroxyethyl-4,5-diaminopyrazole, and salts thereof are preferred; and as the coupler, 2,4-diaminophenoxyethanol, metaminophenol, 2-methyl-5-aminophenol, resorcin, 2-methylresorcin, 4-chlororesorcin, 1-naphthol, 2-amino-3-hydroxypyridine, and 2-amino-4-(8-hydroxyethyl)aminoanisole, and salts thereof are preferred.

In the case of using the dyeing agent other than the component (A), its content in the hair cosmetic is preferably 0.01% by mass or more, and more preferably 0.05% by mass or more, and it is preferably 1% by mass or less, and more preferably 0.5% by mass or less.

(Aqueous Medium)

The hair cosmetic typically contains an aqueous medium. Examples of the aqueous medium include water; a lower alcohol, such as ethanol and isopropyl alcohol; and a low-molecular diol or triol having 6 or less carbon atoms, such as 1,3-butylene glycol, glycerin, ethylene glycol, and propylene glycol, with water being preferred. Although the content of the aqueous medium in the hair cosmetic can be appropriately selected depending upon the formulation of the hair cosmetic, it is typically in a range of 1 to 95% by mass. In the case of using water as the aqueous medium, the content of water in the hair cosmetic is preferably 50% by mass or more, more preferably 60% by mass or more, and still more preferably 70% by mass, and it is preferably 95% by mass or less, and more preferably 90% by mass or less.

<pH>

From the viewpoint of improvement in hair dyeing properties, a pH of the hair cosmetic of the present invention is preferably 8.0 or more, more preferably 8.5 or more, and still more preferably 9.0 or more. This is because the component (A) that is the melanin precursor reacts with oxygen in air under a basic condition, whereby it is liable to be converted into a melanin pigment. From the viewpoint of improvement in hair dyeing properties and suppressing any damage to the hair, the foregoing pH is preferably 12.0 or less, more preferably 11.0 or less, still more preferably 10.5 or less, and yet still more preferably 10.0 or less.

The aforementioned pH is a measured value at 25°C, and specifically, it can be measured by a method described in the section of Examples.

A production method the hair cosmetic of the present invention is not particularly limited. For example, the hair cosmetic of the present invention can be produced by blending the aforementioned respective components by a method described in the section of Examples and mixing the blend by using a known stirring device or the like.

[Dyeing Method of Hair]

The present invention further provides a dyeing method of hair including a step of applying the aforementioned hair cosmetic on hair. For example, in the case where the hair cosmetic is a hair cleansing agent, such as a shampoo, the hair cleansing agent is applied on hair, lathered to cleanse the hair, and then rinsed away with water. In the case where the hair cosmetic is a hair conditioning agent, a hair treatment agent, or a hair dye, the hair cosmetic is applied on hair, optionally allowed to stand for a short time (about 1 to 5 minutes), and then rinsed away with water. By daily repeating such a step, gray hair dyeing can be performed easily and within a short period of time.

From the viewpoint of actually feeling a dyeing effect owing to the hair cosmetic, the amount of the component (A) in the hair cosmetic to be applied on hair is typically 0.001 g or more, preferably 0.01 g or more, and more preferably 0.03 g or more, and from the viewpoint of costs, it is 10 g or less, preferably 5 g or less, and more preferably 1 g or less, relative to 100 g of the hair per week.

With respect to the aforementioned embodiments, the present invention discloses the following.

<1> A hair cosmetic containing the following components (A) and (B):

(A) a compound represented by the following general formula (1) or a salt thereof:

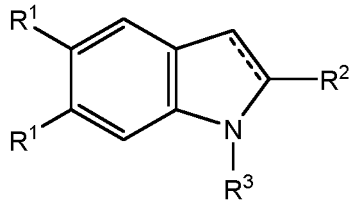

(1)

wherein a broken line represents the presence or absence of a $\pi$ bond; $R^1$ represents a hydroxy group or an acetoxy group; $R^2$ represents a hydrogen atom or -COOR (where R is a hydrogen atom, a methyl group, or an ethyl group); and $R^3$ represents a hydrogen atom, an acetyl group, a methyl group, or an ethyl group, and
(B) at least one polymer selected from the group consisting of polyvinyl alcohol and polyvinylpyrrolidone each having an average degree of polymerization of 700 or more and 10,000 or less.

<2> The hair cosmetic as set forth in the above <1>, wherein the component (A) is one or more selected from the group consisting of 5,6-dihydroxyindole, 5,6-dihydroxyindole-2-carboxylic acid, and 5,6-dihydroxyindoline, and salts thereof.
<3> The hair cosmetic as set forth in the above <1> or <2>, wherein the component (A) is one or two selected from the group consisting of 5,6-dihydroxyindole and 5,6-dihydroxyindole-2-carboxylic acid.
<4> The hair cosmetic as set forth in any of the above <1> to <3>, wherein the component (A) is a combination of 5,6-dihydroxyindole and 5,6-dihydroxyindole-2-carboxylic acid, and a molar ratio of 5,6-dihydroxyindole to 5,6-dihydroxyindole-2-carboxylic acid is 50/50 to 99/1.
<5> The hair cosmetic as set forth in any of the above <1> to <4>, wherein the average degree of polymerization of the component (B) is 700 or more and 7,000 or less.
<6> The hair cosmetic as set forth in any of the above <1> to <5>, wherein the average degree of polymerization of the component (B) is 700 or more and 3,700 or less.
<7> The hair cosmetic as set forth in any of the above <1> to <6>, wherein the average degree of polymerization of the component (B) is 1,000 or more and 2,300 or less.
<8> The hair cosmetic as set forth in any of the above <1> to <7>, wherein the average degree of polymerization of the component (B) is 1,800 or more and 2,300 or less.
<9> The hair cosmetic as set forth in any of the above <1> to <8>, wherein the component (B) is polyvinyl alcohol.
<10> The hair cosmetic as set forth in the above <9>, wherein a degree of saponification of the polyvinyl alcohol is 70% or more and 95% or less.
<11> The hair cosmetic as set forth in any of the above <1> to <10>, wherein the content of the component (A) is

0.01% by mass or more and 40% by mass or less.
<12> The hair cosmetic as set forth in any of the above <1> to <11>, wherein the content of the component (A) is 0.05% by mass or more and 5% by mass or less.
<13> The hair cosmetic as set forth in any of the above <1> to <12>, wherein the content of the component (A) is 0.10% by mass or more and 1% by mass or less.
<14> The hair cosmetic as set forth in any of the above <1> to <13>, wherein the content of the component (B) is 0.05% by mass or more and 5.0% by mass or less.
<15> The hair cosmetic as set forth in any of the above <1> to <14>, wherein the content of the component (B) is 0.20% by mass or more and 1.0% by mass or less.
<16> The hair cosmetic as set forth in any of the above <1> to <15>, wherein the content of the component (B) is 0.25% by mass or more and 0.5% by mass or less.
<17> The hair cosmetic as set forth in any of the above <1> to <16>, wherein a mass ratio (B)/(A) of the component (B) to the component (A) in the hair cosmetic is 0.001 or more and 1,000 or less.
<18> The hair cosmetic as set forth in any of the above <1> to <17>, wherein a mass ratio (B)/(A) of the component (B) to the component (A) in the hair cosmetic is 0.1 or more and 5 or less.
<19> The hair cosmetic as set forth in any of the above <1> to <18>, further containing a surfactant as a component (C).
<20> The hair cosmetic as set forth in the above <19>, wherein the component (C) is a nonionic surfactant.
<21> The hair cosmetic as set forth in the above <20>, wherein the content of the nonionic surfactant is 0.01% by mass or more and 5% by mass or less.
<22> The hair cosmetic as set forth in any of the above <1> to <21>, further containing an alkaline agent.
<23> The hair cosmetic as set forth in the above <22>, wherein the content of the alkaline agent is 0.01% by mass or more and 10% by mass or less.
<24> The hair cosmetic as set forth in any of the above <1> to <23>, having a pH of 8.0 or more and 12.0 or less.
<25> A hair cosmetic containing the following components (A) and (B):

(A) one or more selected from the group consisting of 5,6-dihydroxyindole, 5,6-dihydroxyindole-2-carboxylic acid, and 5,6-dihydroxyindoline, and salts thereof, and
(B) polyvinyl alcohol having an average degree of polymerization of 700 or more and 3,700 or less, wherein the content of the component (A) is 0.05% by mass or more and 5% by mass or less, and the content of the component (B) is 0.20% by mass or more and 1.0% by mass or less, and
a pH is 8.0 or more and 12.0 or less.

<26> A hair cosmetic containing the following components (A) and (B):

(A) one or two selected from the group consisting of 5,6-dihydroxyindole and 5,6-dihydroxyindole-2-carboxylic acid, and
(B) polyvinyl alcohol having an average degree of polymerization of 700 or more and 3,700 or less, wherein the content of the component (A) is 0.05% by mass or more and 5% by mass or less, and the content of the component (B) is 0.20% by mass or more and 1.0% by mass or less, and
a pH is 8.0 or more and 12.0 or less.

<27> A hair cosmetic containing the following components (A) and (B):

(A) one or two selected from the group consisting of 5,6-dihydroxyindole and 5,6-dihydroxyindole-2-carboxylic acid, and
(B) polyvinyl alcohol having an average degree of polymerization of 1,000 or more and 2,300 or less, wherein the content of the component (A) is 0.05% by mass or more and 5% by mass or less, and the content of the component (B) is 0.20% by mass or more and 1.0% by mass or less, and
a pH is 8.0 or more and 12.0 or less.

<28> A hair cosmetic containing the following components (A) and (B):

(A) one or two selected from the group consisting of 5,6-dihydroxyindole and 5,6-dihydroxyindole-2-carboxylic acid, and
(B) polyvinyl alcohol having an average degree of polymerization of 1,800 or more and 2,300 or less, wherein the content of the component (A) is 0.05% by mass or more and 5% by mass or less, and the content of the component (B) is 0.20% by mass or more and 1.0% by mass or less, and
a pH is 8.0 or more and 12.0 or less.

<29> A hair cosmetic containing the following components (A) and (B):

(A) one or more selected from the group consisting of 5,6-dihydroxyindole, 5,6-dihydroxyindole-2-carboxylic acid, and 5,6-dihydroxyindoline, and salts thereof, and
(B) polyvinyl alcohol having an average degree of polymerization of 700 or more and 3,700 or less, wherein a mass ratio (B)/(A) of the component (B) to the component (A) in the hair cosmetic is 0.1 or more and 5 or less, and a pH is 8.0 or more and 12.0 or less.

<30> The hair cosmetic as set forth in any of the above <1> to <29>, which is a hair cleansing agent, a hair rinse, a hair conditioning agent, a hair treatment agent, a hair styling agent, or a hair growth promoter.
<31> The hair cosmetic as set forth in any of the above <1> to <30>, which is a hair cleansing agent.
<32> A dyeing method of hair including a step of applying the hair cosmetic as set forth in any of the above <1> to <31> on hair.

Examples

The present invention is hereunder described by reference to Examples, but it should be construed that the present invention is not limited to the scope of the Examples. In the present Examples, the pH measurement and the preparation and evaluation of hair cosmetic were performed by the following methods.

[pH Measurement]

A pH at 25°C of the hair cosmetic was measured with a pH meter (manufactured by HORIBA, Ltd., main body of pH meter: Portable pH meter D-71, electrode: Micro ToupH electrode 9618S-10D).

[Preparation of Hair Cosmetic (Shampoo)]

Examples 1 to 8 and Comparative Examples 1 to 3

Shampoos each having a composition shown in Table 1 were produced by the following method.
In a 100-mL screwed tube (manufactured by Maruemu Corporation), 44.5 g (active component: 12 g) of an anionic surfactant "EMAL E-27C" (manufactured by Kao Corporation, sodium polyoxyethylene (2) lauryl ether sulfate, active component amount: 27% by mass), 25.8 g of ion-exchanged water, 1.5 g of monoethanolamine, 1.0 g (active component: 0.5 g) of a citric acid aqueous solution (50%), 0.2 g of ascorbic acid, 2.0 g of sodium bicarbonate, and 10 g (active component: 0.3 g) of every polyvinyl alcohol (PVA) aqueous solution (3% by mass) were added and mixed with a magnetic stirrer until the contents became uniform, and then, the solution was deaerated. The deaerated solution was introduced into a glove box filled with nitrogen, and a portion in an amount of 8.5 g based on the whole amount (85.0 g) was charged in a 30-mL screwed tube (manufactured by Maruemu Corporation). 1.5 g of the solution of the component (A) was added thereto and mixed in a nitrogen atmosphere by using a magnetic stirrer until the contents became uniform, thereby obtaining a shampoo for evaluation. All of the shampoos of the examples had a pH of 9.6.

[Evaluation of Hair Dyeing Properties]

A hair bundle of a Chinese gray hair having a mass of 1 g, purchased from Beaulax Co., Ltd. (10 cm/a trade name: Human Gray Hair (100%), trade No.: BM-W-A) was cleansed twice with a plain shampoo having a composition shown below, dried, and then used for the evaluation of hair dyeing properties.

| (Plain Shampoo) | (% by mass) |
|---|---|
| Sodium polyoxyethylene lauryl ether sulfate | |
| (EMAL E-27C (active component amount: 27% by mass), manufactured by Kao Corporation) | 42 |
| Coconut oil fatty acid N-methylethanolamide | |
| (AMINON C-11S, manufactured by Kao Corporation) | 3 |
| Citric acid | 0.2 |
| Methyl paraben | 0.3 |
| Purified water | Remainder |
| Total | 100 |

The hair bundle was rinsed with tap water for 30 seconds; the shampoo obtained in each of the examples was uniformly applied on the rinsed hair bundle such that a bath ratio (hair bundle/water/shampoo) of 1/1/0.1; and the hair bundle was lathered for one minute and then rinsed with tap water for 30 seconds. The hair bundle was dried with a towel and then dried with warm air ejected from a dryer.

Such a hair dyeing treatment with a shampoo was performed 15 times.

Before and after the hair dyeing treatment, a hue (L, a*, b*) of the hair bundle was measured. Using a handy spectrophotometer (manufactured by Nippon Denshoku Industries Co., Ltd., a color difference meter "NF777"), the hue of the hair bundle was measured five times with respect to a position of 2 to 3 cm from the top part of the hair bundle (the side at which the hairs were bundled), and an average value thereof was determined. A color difference ΔE was calculated from the hues before and after hair dyeing according to the following expression (I).

$$\Delta E = \{(L_1 - L_0)^2 + (a^*_1 - a^*_0)^2 + (b^*_1 - b^*_0)^2\}^{0.5} \qquad (I)$$

($L_0$, $a^*_0$, $b^*_0$: Hue before hair dyeing, $L_1$, $a^*_1$, $b^*_1$: Hue after hair dyeing)

Table 1

| | | Example | | | | | | | Comparative Example | | Example | Comparative Example |
|---|---|---|---|---|---|---|---|---|---|---|---|---|
| | | 1 | 2 | 3 | 4 | 5 | 6 | 7 | 1 | 2 | 8 | 3 |
| (A) | (A1) 5,6-Dihydroxyindole solution | 15.0 | | 15.0 | 15.0 | 15.0 | 15.0 | 15.0 | 15.0 | 15.0 | | |
| | (A2) 5,6-Dihydroxyindole solution | | 15.0 | | | | | | | | | |
| | (A3) 5,6-Dihydroxyindoline • HBr solution | | | | | | | | | | 15.0 | 15.0 |
| (B) | (B1) PVA (average degree of polymerization: 2,000, degree of saponification: 79 to 81%) | 0.3 | 0.3 | | | | | | | | 0.3 | |
| | (B2) PVA (average degree of polymerization: 2,500, degree of saponification: 79 to 81%) | | | 0.3 | | | | | | | | |
| | (B3) PVA (average degree of polymerization: 3,500, degree of saponification: 87 to 89%) | | | | 0.3 | | | | | | | |
| | (B4) PVA (average degree of polymerization: 2,400, degree of saponification: 87 to 89%) | | | | | 0.3 | | | | | | |
| | (B5) PVA (average degree of polymerization: 1,700, degree of saponification: 87 to 89%) | | | | | | 0.3 | | | | | |
| | (B6) PVA (average degree of polymerization: 1,000, degree of saponification: 87 to 89%) | | | | | | | 0.3 | | | | |
| (B)' | (B1)' PVA (average degree of polymerization: 500, degree of saponification: 87 to 89%) | | | | | | | | 0.3 | | | |
| Others | Anionic surfactant (sodium polyoxyethylene (2) lauryl ether sulfate: 27%) | 44.5 | 44.5 | 44.5 | 44.5 | 44.5 | 44.5 | 44.5 | 44.5 | 44.5 | 44.5 | 44.5 |
| | Monoethanolamine | 1.5 | 1.5 | 1.5 | 1.5 | 1.5 | 1.5 | 1.5 | 1.5 | 1.5 | 1.5 | 1.5 |
| | Sodium bicarbonate | 2.0 | 2.0 | 2.0 | 2.0 | 2.0 | 2.0 | 2.0 | 2.0 | 2.0 | 2.0 | 2.0 |
| | Citric acid aqueous solution (50%) | 1.0 | 1.0 | 1.0 | 1.0 | 1.0 | 1.0 | 1.0 | 1.0 | 1.0 | 1.0 | 1.0 |
| | Ascorbic acid | 0.2 | 0.2 | 0.2 | 0.2 | 0.2 | 0.2 | 0.2 | 0.2 | 0.2 | 0.2 | 0.2 |
| | Ion-exchanged water | 35.5 | 35.5 | 35.5 | 35.5 | 35.5 | 35.5 | 35.5 | 35.5 | 35.8 | 35.5 | 35.8 |
| Sum total (% by mass) | | 100.0 | 100.0 | 100.0 | 100.0 | 100.0 | 100.0 | 100.0 | 100.0 | 100.0 | 100.0 | 100.0 |
| Mass ratio (B)/(A) | | 1.8 | 2.0 | 1.8 | 1.8 | 1.8 | 1.8 | 1.8 | - | 0 | 2.0 | 0 |
| Evaluation of hair dyeing properties (ΔE) | | 29 | 30 | 26 | 25 | 25 | 27 | 27 | 22 | 18 | 12 | 8 |

The components in the table are shown below. All of the active component amounts (% by mass) described in Table 1 are tangible. Component (A):

(A1) 5,6-Dihydroxyindole solution: a solution produced by the method described in Japanese Patent No. 5570161 (5,6-dihydroxyindole: 1% by mass, 5,6-dihydroxyindole-2-carboxylic acid: 0.14% by mass, ethanol: 20% by mass, water: remainder)
(A2) 5,6-Dihydroxyindole solution: manufactured by Matrix Scientific Inc., 5,6-dihydroxyindole: 1% by mass, ethanol: 20% by mass, water: remainder
(A3) 5,6-Dihydroxyindoline hydrobromide solution: manufactured by AK Scientific, Inc., 5,6-dihydroxyindoline hydrobromide: 1% by mass, ethanol: 20% by mass, water: remainder

Component (B):

(B1) Polyvinyl alcohol (average degree of polymerization: 2,000, degree of saponification: 79 to 81%): "PVA-420", manufactured by Kuraray Co., Ltd.
(B2) Polyvinyl alcohol (average degree of polymerization: 2,500, degree of saponification: 79 to 81%): "JL-25E", manufactured by JAPAN VAM & POVAL CO., LTD.
(B3) Polyvinyl alcohol (average degree of polymerization: 3,500, degree of saponification: 87 to 89%): "PVA-235", manufactured by Kuraray Co., Ltd.
(B4) Polyvinyl alcohol (average degree of polymerization: 2,400, degree of saponification: 87 to 89%): "PVA-224", manufactured by Kuraray Co., Ltd.
(B5) Polyvinyl alcohol (average degree of polymerization: 1,700, degree of saponification: 87 to 89%): "PVA-217", manufactured by Kuraray Co., Ltd.
(B6) Polyvinyl alcohol (average degree of polymerization: 1,000, degree of saponification: 87 to 89%): "PVA-210", manufactured by Kuraray Co., Ltd.

With respect to the average degrees of polymerization of the above (B1) to (B6), those described in the brochures of Kuraray Co., Ltd. and JAPAN VAM & POVAL CO., LTD. were made by reference.
Component (B)':
(B1)': Polyvinyl alcohol (average degree of polymerization: 500, degree of saponification: 87 to 89%): "EG-05", manufactured by The Nippon Synthetic Chemical Industry Co., Ltd.
With respect to the average degree of polymerization of the above (B1)', that described in Japanese Patent No. 5416586 was made by reference.
Anionic Surfactant:
Sodium polyoxyethylene (2) lauryl ether sulfate: "EMAL E-27C", manufactured by Kao Corporation, active component amount: 27% by mass
It is noted from Table 1 that all of the hair cosmetics of the present invention reveal high hair dyeing properties (Examples 1 to 8). In contrast, the hair cosmetic of Comparative Example 1 in which the average degree of polymerization of the component (B) used falls outside the range prescribed in the present application and the hair cosmetics of Comparative Examples 2 and 3 in which the component (B) is not contained are lowered in the hair dyeing properties as compared with the hair cosmetics of the present invention.

Formulation Example 1 (Shampoo Formulation)

A shampoo having a composition shown in Table 2 was produced by the same method as mentioned above, except that in Example 1, a polyvinylpyrrolidone (PVP) aqueous solution (3% by mass) was used in place of the polyvinyl alcohol (PVA) aqueous solution (3% by mass). The shampoo had a pH of 9.6.

Table 2

| | | Formulation Example 1 |
|---|---|---|
| (A) | (A1) 5,6-Dihydroxyindole solution | 15.0 |
| (B) | (B7) PVP | 0.3 |

(continued)

| | | Formulation Example 1 |
|---|---|---|
| Others | Anionic surfactant (sodium polyoxyethylene (2) lauryl ether sulfate: 27%) | 44.5 |
| | Monoethanolamine | 1.5 |
| | Sodium bicarbonate | 2.0 |
| | Citric acid aqueous solution (50%) | 1.0 |
| | Ascorbic acid | 0.2 |
| | Ion-exchanged water | 35.5 |
| Sum total (% by mass) | | 100.0 |

The components in the table are shown below. All of the active component amounts (% by mass) described in Table 2 are tangible.

(A1): 5,6-Dihydroxyindole solution: Same as that mentioned above
(B7): Polyvinylpyrrolidone (average degree of polymerization: 1,500): "Polyvinylpyrrolidone K90", manufactured by Wako Pure Chemical Industries, Ltd.

Anionic Surfactant:
Sodium polyoxyethylene (2) lauryl ether sulfate: "EMAL E-27C", manufactured by Kao Corporation, active component amount: 27% by mass

Industrial Applicability

In accordance with the hair cosmetic of the present invention, it is able to simply obscure gray hair through a daily hair care behavior, such as shampooing, and to dye the hair to a sufficient level by continuous employments.

**Claims**

1. A hair cosmetic comprising the following components (A) and (B):

(A) a compound represented by the following general formula (1) or a salt thereof:

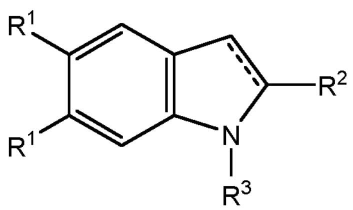

(1)

wherein a broken line represents the presence or absence of a $\pi$ bond; $R^1$ represents a hydroxy group or an acetoxy group; $R^2$ represents a hydrogen atom or -COOR (where R is a hydrogen atom, a methyl group, or an ethyl group); and $R^3$ represents a hydrogen atom, an acetyl group, a methyl group, or an ethyl group, and
(B) at least one polymer selected from the group consisting of polyvinyl alcohol and polyvinylpyrrolidone each having an average degree of polymerization of 700 or more and 10,000 or less.

2. The hair cosmetic according to claim 1, wherein the average degree of polymerization of the component (B) is 700 or more and 7,000 or less.

3. The hair cosmetic according to claim 1 or 2, wherein the content of the component (A) is 0.01% by mass or more and 40% by mass or less.

4. The hair cosmetic according to any one of claims 1 to 3, wherein the content of the component (B) is 0.05% by mass or more and 5.0% by mass or less.

5. The hair cosmetic according to any one of claims 1 to 4, further comprising a surfactant as a component (C).

6. The hair cosmetic according to any one of claims 1 to 5, which is a hair cleansing agent.

7. A dyeing method of hair comprising a step of applying the hair cosmetic according to any one of claims 1 to 6 on hair.

## INTERNATIONAL SEARCH REPORT

International application No.
PCT/JP2018/042680

A. CLASSIFICATION OF SUBJECT MATTER
Int.Cl. A61K8/49(2006.01)i, A61K8/46(2006.01)i, A61K8/81(2006.01)i, A61Q5/02(2006.01)i, A61Q5/10(2006.01)i

According to International Patent Classification (IPC) or to both national classification and IPC

B. FIELDS SEARCHED

Minimum documentation searched (classification system followed by classification symbols)
Int.Cl. A61K8/00-99, A61Q1/00-90/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2003-518029 A (HENKEL KOMMANDITGESELLSCHAFT AUF AKTIEN.) 03 June 2003, paragraphs [0009]-[0015], [0071], [0150]-[0152] & JP 2003-518028 A & JP 2003-525873 A & JP 2004-500361 A & US 2003/0028978 A1, paragraphs [0011]-[0034], [0148], [0303]-[0304] & US 2002/0194684 A1 & US 2003/0033678 A1 & US 2003/0172469 A1 & WO 2001/045655 A1 & DE 19961910 A1 & DE 10059292 A1 & CN 1411363 A | 1-5, 7<br>1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 November 2018 (30.11.2018) | 11 December 2018 (11.12.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/042680

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2014-152145 A (AICELLO CORPORATION) 25 August 2014, claims 1, 4, paragraphs [0022]-[0025] (Family: none) | 1-7 |
| Y | JP 2014-24766 A (REAL CHEMICAL CO., LTD.) 06 February 2014, paragraphs [0001], [0013], [0024], [0031], [0041] (Family: none) | 1-7 |
| Y | JP 2004-525130 A (UNILEVER NV.) 19 August 2004, claims 1-2 & US 2003/0074748 A1, claims 1-2 & US 2003/0051297 A1 & WO 2002/074266 A2 & CA 2440112 A1 & CN 1498100 A | 1-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007326805 A
- JP 1319413 A
- JP 2003342139 A
- JP 5570161 B
- JP 5416586 B